# EUROPEAN PATENT APPLICATION

(11) **EP 0 985 931 A2**
(43) Date of publication of application: **15.03.2000**
(21) Application number: 99115877.5
(22) Date of filing: 12.08.1999
(51) Int. Cl.: G01N 33/571, G01N 33/543, G01N 33/558, C12N 15/30, C07K 14/44

(54) **Recombinant antigen immunoassay for the diagnosis of syphilis**

(30) Priority: 04.09.1998 US 148920
(71) Applicant: Becton, Dickinson and Company, Franklin Lakes, New Jersey 07417 (US)
(72) Inventor: Mullenix, Michael C., Loganville, Georgia 30052 (US); Deutsch, John, Marietta, Georgia 30066 (US)
(74) Representative: Helbing, Jörg, Dr. Dipl.-Chem.

(57) **Abstract**

A method for detecting anti-Treponema pallidum antibody and diagnosing syphilis has been provided. Fusion protein antigens from the fusion of a peptide sequence having an amino acid sequence encoded by the nucleic acid sequence of SEQ. ID. No. 4 to Treponema pallidum membrane proteins are used as antigens in immunoassay of test samples for the presence of anti-Treponema pallidum membrane protein antibodies. A test kit for diagnosing syphilis is also provided comprising a container having therein the fusion protein antigens.

## Description

### FIELD OF THE INVENTION

This invention generally relates to an improved method for diagnosing Treponema pallidum infection by immunoanalysis by utilizing the antigen-antibody reaction between, for example, PP15 and/or PP17 antigen, and anti-Treponema antibody.

### BACKGROUND OF THE INVENTION

The principal method for diagnosis of syphilis has been by immunoassay techniques which utilize the antigen-antibody reaction. Syphilis is caused by Treponema pallidum infection. The immunoassay method for diagnosis of syphilis employs several of the antigens expressed on the Treponema pallidum cell membrane. For example, U.S. Patent No. 4,740,467 discloses a method for diagnosing syphilis by immunoassay detecting the presence of T. pallidum with monoclonal antibodies against a 47 kDa T. pallidum membrane antigen. Japanese Kohyo publication Hei-2-500403 describes producing the 47 kDa membrane antigen protein and immunoassaying anti-Treponema pallidum antibody in patient samples.

Recombinant T. pallidum antigens offer many advantages over the use of conventional T. pallidum cell suspensions. Most of the advantages are related to the high purity of the recombinant T. pallidum antigens. Recombinant antigens utilizing fusion protein expression systems can easily reach purities of >95% after only two simple chromatography procedures. Once purified, the recombinant antigens can be used directly in immunoassays.

Antigens, having molecular weights of 15 kDa and 17 kDa respectively, are both present on the surface of T. pallidum cells. The genes coding for these two proteins have been cloned and their amino acid sequences are known. See, Purcell et al., Infect. And Immunity, 57:3708-3714. (1989). U.S. Patent No. 5,578,456 describes an immunoassay method which utilizes fusion proteins from the fusion of glutathione-S-transferase (GST) to the N-terminal of the 15 kDa (G15) or 17 kDa (G17) T. pallidum membrane antigen. The fusion protein antigens G15 and G17 are employed as antigens to detect the presence of anti-T. pallidum antibody in patient samples.

The need still exists to develop improved methods for diagnosing syphilis. Increased sensitivity and specificity in immunoanalysis can reduce the amount of sample needed for assay and thus decrease the interfering effect of non-antibody materials in the sample. The present invention describes improved antigens and methods for diagnosing syphilis.

### SUMMARY OF THE INVENTION

The present invention provides an immunoassay method for detecting anti-Treponema pallidum antibody. The method comprises (1) expressing T. pallidum antigens in an E. coli expression vector utilizing a biotinylated fusion protein (from, for example, the PinPoint Vector Sequence); and (2) immunoanalyzing a sample for reaction between said fusion protein antigens and anti-T. pallidum antibody in the sample. The method can be used for the diagnosis of syphilis with improved sensitivity and specificity over the methods in the prior art.

Syphilis infections are commonly diagnosed by the detection of T. pallidum specific antibodies in patient serum. There are two basic categories of Syphilis immunoassays. Immunoassays in the first category are inexpensive and take advantage of the cross reactivity of T. pallidum specific antibodies with cardiolipin. The RPR (Rapid Plasma Region) and VDRL (Venereal Disease Research Laboratory) tests are examples of the non-Treponemal immunoassays in this first category. RPR and VDRL tests suffer from false positive reactions with sera from several other disease states and are used primarily as screening assays. The second category of Syphilis immunoassays utilizes whole T. pallidum cells for detection of specific antibodies in patient serum. FTA-ABS (Fluorescent Treponemal Antibody Absorption) and MHA-TP (Microhemagglutination-Treponema pallidum) are examples of Treponemal immunoassays in the second category. The Treponemal immunoassays are sensitive, highly specific and expensive. Treponemal tests are typically used for confirmatory testing. The development of recombinant T. pallidum antigens is leading to new tests with both screening and confirmatory applications. The recombinant antigen assays combine the advantages of both Treponemal and non-Treponemal assays.

The PinPoint™ (Promega) E. coli vector system is designed to simplify the cloning, expression and purification of recombinant proteins. In the PinPoint™ expression system target proteins are genetically fused to a peptide sequence that is biotinylated in vivo. The biotinylated peptide allows purification of the recombinant protein through monomeric avidin affinity chromatography. Several other manufacturers (Univ. of Texas, Fuji Rebio, BioKit) produce similar antigens expressed in the GST (Glutathione S-Transferase) gene fusion vector (Pharmacia). The immunodiagnostic use of the GST fusion antigens is patented under U.S. Patent No. 5,578,456. The "PinPoint" ("PP") fusion antigens described herein offer several advantages over the GST fusion antigens.

The first advantage of the PP fusion antigens described in the present invention is the higher level of immunoreactivity observed when compared to GST antigens on a weight basis. In microwell immunoassays, higher concentrations of GST antigens are required to provide the same level of reactivity as with PP antigens. This observation is significant because of its implications for assay sensitivity and specificity. Use of the PP antigens will improve immunoassay sensitivity because more reactivity can be incorporated in the assay without adding more total antigen.

A second advantage to the PP expressed T. pallidum antigens is the smaller size of the fusion protein. The GST system fusion peptide adds 27 kDa to the mass of the target antigen while the PP system fusion peptide only adds 13 kDa to the mass of the target. In the PP system, the fusion protein makes up 46% of the 15 kDa antigen's total mass and 43% of the 17 kDa antigen's total mass. In the GST system, the fusion protein makes up 64% of the 15 kDa antigen's total mass and 61% of the 17 kDa antigen's total mass. The smaller size of the fusion protein sequence in the PP system may reduce the possibility of fusion protein steric hindrance of specific antibody recognition of the T. pallidum antigen sequence.

The in vivo biotinylation of the fusion protein in the PP system also provides several advantages over the GST system. The biotinylation allows the use of monomeric avidin affinity chromatography to purify the recombinant PP proteins. Binding of monomeric avidin is reversible and biotinylated proteins are easily eluted from monomeric avidin resins with free biotin.

The high affinity interaction of biotinylated antigens with native avidin (or streptavidin) provides advantages when chemical labeling of the recombinant antigen is required. The nearly irreversible binding of native avidin to biotin provides several options for chemical labeling. Avidin can be chemically labeled by any of several common approaches (colloidal gold, enzymes, fluorescein, etc). Once the avidin is labeled, it can simply be mixed with the PP protein. The labeled avidin will bind biotinylated PP protein and thereby provide a chemical label for the recombinant protein.

Native avidin is made up of four identical subunits. Each subunit is capable of binding a single biotin molecule. The multivalent nature of avidin allows it to be used to increase the amount of PP proteins immobilized on a solid phase. For example, pre-coating multi-well plates with avidin will increase the amount of PP antigen that can captured on the plate surface. An additional advantage to this approach is that the antigenic epitopes on the recombinant protein are presented without the steric hindrance that is often observed when coating antigens directly to the micro-well plates.

This invention further relates to a test kit for diagnosing syphilis comprising a compartmentalized container having therein in close confinement one or more container means, one of which contains at least one of the fusion protein antigens described above.

### BRIEF DESCRIPTION OF THE FIGURES

Figure 1 is a graph showing the results of ELISA testing in accordance with the method of the present invention using PP15 and PP17 as antigens in comparison with the results of the comparable assay using GST15, GST17 and GST47.
Figure 2 is a graph showing the results of ELISA testing using various dilutions of test serum in accordance with the method of the present invention.
Figure 3 is a graph showing the results of ELISA testing which depicts the signal to noise (S/N) ratio tin 37 different Treponema positive serum samples contacted with two kinds of recombinant 15 kDa T. pallidum antigens.
Figure 4 is a graph showing the results of ELISA testing which depicts the S/N ratio for 21 different Treponema negative serum samples contacted with two recombinant 15 kDa T. pallidum antigens.
Figure 5 is a graph showing the results of ELISA testing which depicts the S/N ratio for 37 different Treponema positive serum samples contacted with two recombinant 17 kDa T. pallidum antigens.
Figure 6 is a graph showing the results of ELISA testing which depicts the S/N ratio for 21 different Treponema negative serum samples contacted with two recombinant 17 kDa T. pallidum antigens.
Figure 7 is a graph showing the results of ELISA testing which depicts the signal to noise (S/N) ratio for 37 different Treponema positive serum samples contacted with a recombinant 47 kDa T. pallidum antigen.
Figure 8 is a graph showing the results of ELISA testing which depicts the S/N ratio for 21 different Treponema negative serum samples contacted with a recombinant 47 kDa T. pallidum antigen.
Figure 9 depicts the Pin Point™ Xa-1 T-Vector.

### DETAILED DESCRIPTION OF THE INVENTION

The present invention provides a method for detecting anti-T. pallidum antibody. This invention is based on the discovery of an immunoassay with substantially higher sensitivity than immunoassays utilizing the G15 or G17 antigens disclosed in the U.S. Patent No. 5,578,456. The novel immunoassay detects anti-T. pallidum antibody using recombinant T. pallidum membrane antigens expressed as fusion proteins containing peptide sequence that undergoes in vivo biotinylation (PP15 and PP17 Sequences). The method of the present invention can be used for diagnosis of syphilis. This invention further provides a diagnostic kit for diagnosing syphilis comprising a container having therein at least one of the fusion protein antigens.

The samples to be tested with the present invention can be from human, animal, or from the secretions of cultured cells. They can be natural body fluids, diluted solutions, or in a solid form. An example of a solid form is a lyophilized powder which must then be dissolved into solution before use. Test samples would ordinarily be human or animal body fluids such as serum or blood.

It is generally known to a person skilled in the art that T. pallidum has several membrane antigen proteins on its cell surface. Any one of these proteins can be used for this invention so long as the DNA sequence encoding the protein is known and thus the protein can be genetically engineered. Examples of these protein antigens include, but are not limited to, the 15 kDa protein, the 17 kDa Protein, and the 47 kDa protein. See, Japanese Kohyo publication Hei-2-500403; Purcell et al., *Infect. And Immunity,* 57:3708-3714 (1989); U.S. Patent No. 5,578,456; Journal of Immunology, 129: 1287-1291 (1982); Journal of Clinical Microbiology, 21:82-87 (1985); Journal of Clinical Microbiology, 30: 115-122 (1992). The genes coding for these three proteins have been cloned and their amino acid sequences have been established. The biotinylation peptide sequence can be fused to the membrane antigens by generally known genetic engineering methods.

In a preferred embodiment, the peptide sequence can be the amino acid sequence of SEQ. ID. No. 5 encoded by the nucleic acid sequence of SEQ. ID. No. 4 wherein this DNA sequence is synthesized or is an isolated DNA sequence encoding the same amino acid sequence. It is further noted that this SEQ. ID. No. 4 is intended to include conservative changes to the nucleic acid sequence which would result in conservative changes to the amino acid sequence of SEQ. ID. No. 5 encoded by this nucleic acid sequence. As is obvious to a person skilled in the art, the DNA sequence encoding the heterologous peptide sequence can be easily linked to the DNA sequence encoding the membrane protein antigens. The DNA linking can be done by PCR or ligation. See Sambrook et al. Molecular Cloning, A Laboratory Manual (2d. Ed., 1989). The DNA sequences can be linked and then subcloned into an expression vector. Alternatively, the two sequences can be subcloned sequentially into an expression vector. The method for expressing proteins in E. coli is generally known in the art. Any suitable expression vector can be used.

Preferably, the expression vector known as the PinPoint™ vector having SEQ. ID. No. 1 can be used herein since a DNA sequence encoding the heterologous peptide sequence has already been cloned into the vector by its commercial supplier (Promega Corp). The DNA sequence is located at the polylinker cloning site indicated by the rectangular box on the right side of the PinPoint vector. See Fig. 9.

The expression of fusion protein antigens such as PP15 and PP17 is achieved by transforming the expression constructs into E. coli. The method for transformation is generally known a in the art. When expressing the fusion proteins, the transformed E. coli cells are cultured under normal growth conditions generally known in the art. Preferably, free biotin is also added to the culture medium. The addition of biotin allows the heterologous peptide sequence in the fusion proteins to be biotinylated in E. coli. Isopropyl-β-D-Thiogalactopyranoside (IPTG) is added to the medium to induce expression of the protein. The biotinylated fusion proteins can then be easily purified by monomeric avidin affinity chromatography, which is known to a person skilled in the art. Thus, the biotinylation in this invention simplifies the purification of the fusion proteins made in E. coli. The prepared fusion protein antigens can be used in an immunoassay procedure. The immunoassay procedure can be any immunoassay procedure known to a person skilled in the art.

In a preferred embodiment, PP15 and/or PP17 are the recombinant antigens of choice for an immunoassay of the present invention. PP15 is encoded by the nucleic acid sequence of SEQ. ID. No. 2 and has the amino acid sequence of SEQ. ID. No. 3. PP15 is a fusion protein from the fusion of a peptide sequence of SEQ. ID. No. 5 which is encoded by the nucleic acid sequence of SEQ. ID No. 4, and T. pallidum membrane protein having a molecular weight of 15 kDa. PP17 is encoded by the nucleic acid sequence of SEQ. ID. No. 6 and has the amino acid sequence of SEQ. ID. No. 7. PP17 is a fusion protein from the fusion of a peptide sequence of SEQ. ID. No. 5 which is encoded by the nucleic acid sequence of SEQ. ID. No. 4, and T. pallidum membrane protein having a molecular weight of 17 kDa.

With respect to the immunoassay procedures referred to above, in one example, the conventional sandwich ELISA procedure can be used wherein the fusion protein antigens are bounded to a solid phase carrier. For speedy assay of multiple samples, a multi-well container can be used. In conducting such an assay, the wells of a multiwell container, such as a 96 well microtiter plate, are coated with one or more fusion protein antigens. The wells having the immobilized proteins are then treated with blocking agents to block the non-specific adhesion sites, and are washed to remove excess free reagents. The test samples are added to the wells and are incubated for reaction between the fusion protein antigen(s) and the anti-T. pallidum antibody, if any is present in the samples. After the incubation, the wells are washed to remove any unbounded micromolecules. A labeled anti-human immunoglobulin G (IgG) and M (IgM) antibody is then added for reaction with any human antibody that is bound to the immobilized protein antigens. After incubation, the wells are again washed to remove any free anti-human IgG and IgM antibody. Typically, the anti-human IgG and IgM antibody is labeled for qualitative or quantitative assay by an enzyme in which case a substrate for the conjugated enzyme is added to the wells for enzymatic reaction. The enzyme reaction produces color changes which indirectly indicate the amount of anti-Treponema pallidum antibodies present in a sample and can be quantified with a spectrometer.

The anti-human IgG and IgM antibody can also be labeled with a radioactive isotope and thus the quantitative measurement will be by a radiation counter. The anti-human IgG and IgM antibody can also be labeled by fluorescence dyes and the anti-T. pallidum antibody, and the sample can be quantified by measuring the intensity of the fluorescence light emitted from the bound anti-human IgG and IgM antibody. Other reagents that can be used in labeling the anti-human IgG and IgM include, but not limited to, formazan (U.S. Patent No. 4,786,589), chromoprotein (U.S. Patent No. 4,302,536), water-dispersible hydrophobic dyes or pigments (U.S. Patent No. 4,373,932).

Another example of a suitable method of immunoassay is the agglutination test, which is also known in the art. Unlike in the sandwich method, a fusion protein antigen is immobilized to particulate carriers. Thus when they are admixed with a testing sample, because the antigen specific antibodies present in the sample will be bivalent, the reaction between the antibodies and the antigens immobilized on the surface of the particulate carriers will cause agglutination of the particles. This reaction can be qualitatively or quantitatively detected.

The immunoassay can also be conducted by an electrochemical enzymatic complementation method in a like manner as in U.S. Patent No. 5,427,912, which is herein incorporated by reference.

It should be understood that any other immunoassay procedures may be used, so long as the reaction between the fusion protein antigens and the corresponding antibodies can be specifically detected. For example, solid phase immunoassay procedures such as those described in U.S. Patent No. 4,703,017 and U.S. Patent No. 5,591,645 are herein incorporated by reference.

Thus such a solid phase immunoassay can utilize the PP15 antigen, the PP17 antigen or both of these antigens, as binder immobilized on the solid phase and further utilizing a visible tracer to determine the presence of anti-Treponema pallidum antibody where the visible tracer is a ligand labeled with a visible particulate label, and further, where the tracer can bind the PP15 antigen or the anti-Treponema pallidum antibody. The visible particulate label can be a sac including a visible dye, and the sac can be a microcapsule or a liposome. The visible particulate label can also be a colored particle or a hydrophobic dye, as described in US. Patent No. 4,703,017. The tracer can be a labeled form of the antibody, or a labeled form of antigen to the antibody.

The solid phase assay can also be a solid phase chromatographic immunoassay, wherein said immunoassay is a device comprising at least a first portion and a second portion, and further a tracer portion which can be contained in the first portion or is a separate portion of the device; wherein said portions are in a lateral configuration to permit capillary flow communication between each portion; said first portion being the portion for application; said tracer portion having said tracer movably supported thereon; and said second portion being the portion for determining the presence of the visible tracer after the sample has flowed to said second portion. The label utilized in said assay can be a label as described above.

Furthermore, it should also be understood that any one of the fusion protein antigens can be used individually or in combination with other fusion protein antigens of this invention. In addition, they could also be used in combination with any other T. pallidum antigens taught in the prior art. These other antigenic proteins include, but are not limited to, purified T. pallidum membrane protein antigens, and the G15 and G17 antigens disclosed in U.S. Patent No. 5,578,456.

The present invention further relates to a test kit for diagnosis of syphilis. The kit comprises a container having therein at least one of the fusion proteins of this invention for use in an immunoassay in accordance with this invention. The fusion protein antigens can be in known concentrations in aqueous solutions. They can also be in lyophilized form to facilitate shipment, and thus the user must first dissolve them before use. The fusion protein antigens can also be covalently bound to a carrier. Examples of such a carrier include but are not limited to, a membrane or film which is preferably porous and capillary as disclosed in U.S. Patent No. 4,168,146, and a multi-well container such as a 96 well microtiter plate. When the fusion protein antigens are bound to a carrier, preferably the carrier is covered by means to protect the antigens, such as a sterilized paper attached to the carrier.

In addition to the fusion protein antigens, the kit in accordance to this invention may, optionally, also have other reagents or devices necessary for immunoassay in a particular method as described herein above. For example, in the sandwich ELISA method as described above, the washing solutions, the blocking agents, the enzyme-labeled anti-human IgG, and the substrate for the enzyme may all be included in the kit. Furthermore, the kit may also contain user instructions for the diagnostic kit. The directions may be printed on sheets such as paper or on the containers of the kit.

Thus, a method for detecting anti-Treponema pallidum antibody and diagnosing syphilis are provided herein. A fusion protein containing heterologous peptide sequence fused to a Treponema pallidum membrane protein antigen, such as the 15 kDa , 17 kDa, or 47 kDa membrane protein is used as antigen in the immunoassay. A diagnostic kit for diagnosing syphilis is also provided comprising a container having therein at least one of the fusion protein antigens disclosed herein.

### EXAMPLES

### Example 1. Recombinant Antigen Titrations

Separate serial dilutions of recombinant T. pallidum antigens were immobilized on plastic micro-well assay plates (Dynatech, immunlon 4). The immobilized antigens included PinPoint™ and GST(BioKit) fusion proteins corresponding to the 15 and 17 kDa membrane antigens of T. pallidum. Serial dilutions of a recombinant GST fusion protein (Capricorn) corresponding to the 47 kDa membrane antigen of T. pallidum were also immobilized to provide a positive control. The plates were washed and blocked with 1% Casein, 0.1 % Tween-20 (trade name) in phosphate buffered saline (assay buffer). A human Syphilis positive serum sample (CDC Non-Treponemal serum panel) was diluted 1:50 in assay buffer and 50 µl were added to each well on the microwell assay plate and incubated for 30 minutes at 37° C. The wells were then washed and 50 ul of peroxidase conjugated anti-human IgG (H+L) solution was added to each well. The plate was incubated for 30 min at 37° C and washed. Fifty µl of TMB substrate was added to each well arid incubated for color development. The reaction was stopped by adding 50 µl of hydrochloric acid solution to each well. The absorbance at 450 nm was determined for each well with a spectrophotometer (Bio-Tek EL312e). The results are shown in Figure 1.

Figure 1 shows the immuno-reactivity of the syphilis positive serum is greater with the recombinant antigens prepared in the PinPoint gene fusion system. Approximately 8-fold less PP15 antigen is required to provide the same level of reactivity as the GST15 antigen and approximately 2-fold less PP17 antigen is required to provide the same level of reactivity as the GST17 antigen. The results demonstrate that significantly higher assay sensitivity can be obtained by utilizing PP15 and PP17 antigens

### Example 2. Titration of Syphilis Positive Antiserum

Recombinant T. pallidum antigens were diluted to a final concentration of 5 µg/ml. Fifty microliter volumes of the antigen solutions were used to immobilize the antigens separately on micro-well assay plates. The plates were washed and blocked with 1% Casein, 0.1% Tween-20 (trade name) in phosphate buffered saline (assay buffer). A human Syphilis positive serum sample (CDC Non-Treponemal serum panel, was serially diluted from 1:10 in assay buffer. Fifty µl of each dilution was added in sequence to separate wells containing the recombinant T. pallidum antigens. The micro-well assay plate was incubated for 30 minutes at 37° C and washed. Fifty µl of peroxidase conjugated anti-human IgG (H+L) solution was added to each well and the plate was incubated for 30 minutes at 37° C. The plate was washed and 50 µl of TMB substrate was added to each well and incubated for color development. The reaction was stopped by adding 50 µl of hydrochloric acid solution to each well. The absorbance at 450 nm was determined for each well with a specrophotometer (Bio-Tek EL312e). The results are shown in Figure 2.

Figure 2 demonstrates that the immuno-reactivity of the syphilis positive serum is greater with the recombinant antigens prepared in the PinPoint gene fusion system. Approximately 2-fold less antisera provides the same level of reactivity with the PP15 antigen when compared to the GST15 antigen. Less antisera is required in assays utilizing the PP17 antigen as compared to assays utilizing the GST17 antigen and higher overall absorbance values are obtained with the PP17 antigen showing that more specific antibody binds. The results demonstrate that higher assay sensitivity can be obtained by utilizing PP15 and PP17 antigens.

### Example 3. Serum Panel Reactivity Comparison of Recombinant T. pallidum Antigens

Recombinant T. pallidum antigens were diluted to 1 µg/ml (PP15, PP17) and 5 µg/ml (GST15, GST17, GST47). Fifty µl of each antigen solution were immobilized separately in individual wells on micro-well assay plates. The plates were washed and blocked with 1% Casein, 0.1% Tween-20 (trade name) in phosphate buffered saline (assay buffer). Treponemal and non-Treponemal syphilis panel serum samples (CDC Atlanta, GA) were diluted 1:50 in assay buffer and 50 µl of each was added to separate micro-wells each containing one of the 5 recombinant T. pallidum antigens. The micro-well assay plates were incubated for 30 minutes at 37° C and washed. Fifty µl of peroxidase conjugated anti-human IgG (H+L) solution was added to each well and the plates were incubated for 30 minutes at 37° C. The plates were washed and 50 µl of TMB substrate was added to each well and incubated for color development. The reaction was stopped by adding 50 µl of hydrochloric acid solution to each well. The absorbance at 450 nm was determined for each well with a spectrophotometer (Bio-Tek EL312e). The signal to noise ratio for each serum sample was determined by dividing the absorbance of each serum sample by the average absorbance of the negative serum samples included in the panel. The results are shown in Figures 3 through 8.

The reactivity of positive and negative syphilis serum samples with recombinant antigens corresponding to the 15 kDa membrane antigen of T. pallidum is shown in Figures 3 and 4. Figure 3 represents the reactivity of 37 different syphilis positive serum samples (verified by conventional method) with the two recombinant 15 kDa antigens. Figure 4 represents the reactivity of 21 different syphilis negative serum samples (verified by conventional methods) with the two recombinant 15 kDa antigens. Similar signal to noise ratios are observed between the two antigens in both the positive and negative samples. The PP15 assay incorporates 5 fold less recombinant antigen than the GST15 assay. The results again demonstrate the immuno-reactivity of the PP15 antigen is greater than the immuno-reactivity of the GST15 antigen. A similar level of immuno-reactivity can be obtained using less total antigen in the PP15 assay, therefore the sensitivity of assays incorporating the PP15 antigen would be greater than assays incorporating the GST15 antigen.

The reactivity of positive and negative syphilis serum samples with recombinant antigens corresponding to the 17 kDa membrane antigen of T. pallidum is shown in Figures 5 and 6. Figure 5 represents the reactivity of 37 different syphilis positive serum samples (verified by conventional method) with the two recombinant 17 kDa antigens. Figure 6 represents the reactivity of 21 different syphilis negative serum samples (verified by conventional methods) with the two recombinant 17 kDa antigens. Similar signal to noise ratios are observed between the two antigens in both the positive and negative samples. The PP17 assay incorporates 5 fold less recombinant antigen than the GST17 assay. The results again demonstrate the immunoreactivity of the PP17 antigen is greater than the immuno-reactivity of the GST17 antigen. The same level of immuno-reactivity can be obtained using less total antigen in the PP17 assay, therefore the sensitivity of assays incorporating the PP17 antigen would be greater than assays incorporating the GST17 antigen. It should also be noted that patient serum immunoreactivity is generally greater with the recombinant antigens corresponding to the 17 kDa antigen as compared to the antigens corresponding to the 15 kDa antigen (compare Figures 3 and 5).

For comparison purposes, the reactivity of positive and negative syphilis serum samples with a recombinant GST fusion antigen corresponding to the 47 kDa membrane antigen of T. pallidum is shown in figures 7 and 8. Figure 7 represents the reactivity of 37 different syphilis positive serum (verified by conventional method) with the recombinant 47 kDa antigen. Figure 8 represents the reactivity of 21 different syphilis negative serum samples (verified by conventional methods) with the recombinant 47 kDa antigen. Figure 7 shows that two positive samples (#5 and #11) fail to immuno-react with the 47 kDa antigen. In addition the overall immuno-reactivity with the 47 kDa antigen is less than that of the recombinant 15 and 17 kDa antigens.

All publications and patent applications mentioned in the specification are indicative of the level of those skilled in the art 10 which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

## Claims

1. A method for determining the presence of anti-Treponema pallidum antibody which comprises assaying anti-Treponema pallidum antibody in a sample by immunoanalysis utilizing the antigen-antibody reaction between PP15 antigen, which is a protein from the fusion of a peptide sequence having an amino acid sequence encoded by the nucleic acid sequence of SEQ. ID. No. 4 to a Treponema pallidum membrane antigen having a molecular weight of 15 kDa, or PP17 antigen, which is a protein from the fusion of a peptide sequence having an amino acid sequence encoded by the nucleic acid sequence of SEQ.ID. No. 4 to a Treponema pallidum membrane antigen having a molecular weight of 17 kDa, or both antigens, and anti-Treponema pallidum antibody in the sample, wherein said antigen is present in a limiting amount in said assay.

2. The method for assay according to Claim 1, wherein the assay is performed by enzyme immunoassay or by agglutination immunoassay.

3. The method for assay according to Claim 1, wherein the assay is performed by solid phase immunoassay using said PP15 antigen, said PP17 antigen or both of said antigens, as binder immobilized on said solid phase and further using a visible tracer to determine the presence of anti-Treponema pallidum antibody wherein said visible tracer is a ligand labeled with a visible particulate label, and further wherein said tracer binds to said PP15 antigen or said anti-Treponema pallidum antibody.

4. The method for assay according to Claim 3, wherein the assay is a solid phase chromatographic immunoassay, and further wherein said immunoassay is a device comprising at least a first portion and a second portion, and further a tracer portion which can be contained in the first portion or is a separate portion of the device;
wherein said portions are in a lateral configuration to permit capillary flow communication between each portion:
said first portion being the portion for application:
said tracer portion having said tracer movably supported thereon; and
said second portion being the portion for determining the presence of the visible tracer after the sample has flowed to said second portion.

5. A method for detecting the presence of anti- Treponema pallidum antibody which comprises assaying anti-Treponema pallidum antibody in a sample by utilizing the antigen-antibody reaction between at least one fusion protein antigen from the fusion of a peptide sequence having an amino acid sequence encoded by the nucleic acid sequence SEQ ID NO. 4 to a Treponema pallidum membrane protein antigen, and anti-Treponema pallidum antibody in the sample, wherein said antigen is present in a limiting amount in said assay.

6. A test kit for diagnosing syphilis comprising a container being compartmentalized and having in close confinement therein PP15 antigen, which is a protein from the fusion of a peptide sequence having an amino acid sequence encoded by the nucleic acid sequence of SEQ. ID. No. 4 to a Treponema pallidum membrane antigen having a molecular weight of 15 kDa, or PP17 antigen, which is a protein from the fusion of a peptide sequence having an amino acid sequence encoded by the nucleic acid sequence of SEQ. ID No. 4 to a Treponema pallidum membrane antigen having a molecular weight of 17 kDa, or both antigens.

7. A test kit for diagnosing syphilis comprising a container being compartmentalized having in close confinement therein
a) at least one fusion protein antigen from the fusion of a peptide sequence having an amino acid sequence encoded by the nucleic acid sequence of SEQ ID NO. 4 to a Treponema pallidum membrane protein antigen:
b) other immunochemical reagents for immunoanalysis of anti-Treponema pallidum antibody; and
c) directions for use of said test kit.

8. An isolated nucleic acid sequence selected from the group consisting of nucleic acid sequences encoding SEQ. ID. No. 4: nucleic acid sequences encoding amino acids 1-257 of SEQ. ID. No.2: and nucleic acid sequences encoding amino acids 1-267 of SEQ. ID. No. 6.

9. An isolated recombinant antigen which is a fusion protein having the amino acid sequence of SEQ. ID. No. 3, or an isolated recombinant antigen which is a fusion protein having the amino acid sequence of SEQ. ID. No. 7.

10. A method for producing a recombinant Treponema pallidum antigen, comprising:
a) transforming a host cell with a recombinant vector having a nucleic acid sequence of SEQ. ID. No. 1, wherein said vector encodes a fusion protein which is an affinity purification tag, and further wherein said vector has the nucleic acid sequence for a T. pallidum 15 kDa membrane protein antigen or 17 kDa membrane protein antigen;
b) culturing said transformed host cell in a culture medium under conditions whereby the recombinant antigen is expressed by the transformed host cell; and
c) purifying said recombinant antigen by means of the affinity purification tag.


SEQUENCE LISTING

<110> Becton, Dickinson and Company

<120> Recombinant Antigen Immunoassay For The Diagnosis of
      Syphilis

<130> 991830ep/JH/ml

<140> 99115877.5
<141> 1999-08-12

<160> 7

<170> PatentIn Ver. 2.1

<210> 1
<211> 3331
<212> DNA
<213> Treponema pallidum

<400> 1
atgaaactga aggtaacagt caacggcact gcgtatgacg ttgacgttga cgtcgacaag 60
tcacacgaaa acccgatggg caccatcctg ttcggcggcg gcaccggcgg cgcgccggca 120
ccggcagcag gtggcgcagg cgccggtaag gccggagagg gcgagattcc cgctccgctg 180
gccggcaccg tctccaagat cctcgtgaag gagggtgaca cggtcaaggc tggtcagacc 240
gtgctcgttc tcgaggccat gaagatggag accgagatca acgctcccac cgacggcaag 300
gtcgagaagg tcctggtcaa ggagcgtgac gcggtgcagg gcggtcaggg tctcatcaag 360
atcggggatc tcgagctcat cgaaggtcgc gaagcttcag ctgggatccg gtaccgatat 420
cagatctccc ggggcggccg cgatctggtt ctatagtgtc acctaaatcg tatgtgtatg 480
atacataagg ttatgtatta attgtagccg cgttctaacg acaatatgtc catatggtgc 540
actctcagta caatctgctc tgatgccgca tagttaagcc agccccgaca cccgccaaca 600
cccgctgacg cgccctgacg ggcttgtctg ctcccggcat ccgcttacag acaagctgtg 660
accgtctccg ggagctgcat gtgtcagagg ttttcaccgt catcaccgaa acgcgcgaga 720
cgaaagggcc tcgtgatacg cctattttta taggttaatg tcatgataat aatggtttct 780
tagacgtcag gtggcacttt tcggggaaat gtgcgcggaa cccctatttg tttatttttc 840
taaatacatt caaatatgta tccgctcatg agacaataac cctgataaat gcttcaataa 900
tattgaaaaa ggaagagtat gagtattcaa catttccgtg tcgcccttat tccctttttt 960
gcggcatttt gccttcctgt ttttgctcac ccagaaacgc tggtgaaagt aaaagatgct 1020
gaagatcagt tgggtgcacg agtgggttac atcgaactgg atctcaacag cggtaagatc 1080
cttgagagtt ttcgccccga agaacgtttt ccaatgatga gcacttttaa agttctgcta 1140
tgtggcgcgg tattatcccg tattgacgcc gggcaagagc aactcggtcg ccgcatacac 1200
tattctcaga atgacttggt tgagtactca ccagtcacag aaaagcatct tacggatggc 1260
atgacagtaa gagaattatg cagtgctgcc ataaccatga gtgataacac tgcggccaac 1320
ttacttctga caacgatcgg aggaccgaag gagctaaccg cttttttgca caacatgggg 1380
gatcatgtaa ctcgccttga tcgttgggaa ccggagctga atgaagccat accaaacgac 1440
gagcgtgaca ccacgatgcc tgtagcaatg gcaacaacgt tgcgcaaact attaactggc 1500
gaactactta ctctagcttc ccggcaacaa ttaatagact ggatggaggc ggataaagtt 1560
gcaggaccac ttctgcgctc ggcccttccg gctggctggt ttattgctga taaatctgga 1620
gccggtgagc gtgggtctcg cggtatcatt gcagcactgg ggccagatgg taagccctcc 1680
cgtatcgtag ttatctacac gacggggagt caggcaacta tggatgaacg aaatagacag 1740
atcgctgaga taggtgcctc actgattaag cattggtaac tgtcagacca agtttactca 1800
tatatacttt agattgattt aaaacttcat ttttaattta aaaggatcta ggtgaagatc 1860
ctttttgata atctcatgac caaaatccct taacgtgagt tttcgttcca ctgagcgtca 1920
gaccccgtag aaaagatcaa aggatcttct tgagatcctt tttttctgcg cgtaatctgc 1980

tgcttgcaaa caaaaaaacc accgctacca gcggtggttt gtttgccgga tcaagagcta 2040
ccaactcttt ttccgaaggt aactggcttc agcagagcgc agataccaaa tactgtcctt 2100
ctagtgtagc cgtagttagg ccaccacttc aagaactctg tagcaccgcc tacatacctc 2160
gctctgctaa tcctgttacc agtggctgct gccagtggcg ataagtcgtg tcttaccggg 2220
ttggactcaa gacgatagtt accggataag gcgcagcggt cgggctgaac ggggggttcg 2280
tgcacacagc ccagcttgga gcgaacgacc tacaccgaac tgagatacct acagcgtgag 2340
cattgagaaa gcgccacgct tcccgaaggg agaaaggcgg acaggtatcc ggtaagcggc 2400
agggtcggaa caggagagcg cacgagggag cttccagggg gaaacgcctg gtatctttat 2460
agtcctgtcg ggtttcgcca cctctgactt gagcgtcgat ttttgtgatg ctcgtcaggg 2520
gggcggagcc tatggaaaaa cgccagcaac gcggcctttt tacggttcct ggccttttgc 2580
tggccttttg ctcacatgtt ctttcctgcg ttatcccctg attctgtgga taaccgtatt 2640
accgcctttg agtgagctga taccgctcgc cgcagccgaa cgaccgagcg cagcgagtca 2700
gtgagcgagg aagcggaaga gcgcccaata cgcaaaccgc ctctccccgc gcgttggccg 2760
attcattaat gcaggttaac ctggcttatc gaaattaata cgactcacta tagggagacc 2820
ggcctcgagc agcaaggaga tggcgcccaa cagtcccccg gccacggggc ctgccaccat 2880
acccacgccg aaacaagcgc tcatgagccc gaagtggcga gcccgatctt ccccatcggt 2940
gatgtcggcg atataggcgc cagcaaccgc acctgtggcg ccggtgatgc cggccacgat 3000
gcgtccggcg tagaggatcg atccgggctt atcgactgca cggtgcacca atgcttctgg 3060
cgtcaggcag ccatcggaag ctgtggtatg gctgtgcagg tcgtaaatca ctgcataatt 3120
cgtgtcgctc aaggcgcact cccgttctgg ataatgtttt ttgcgccgac atcataacgg 3180
ttctggcaaa tattctgaaa tgagctgttg acaattaatc atcggctcgt ataatgtgtg 3240
gaattgtgag cggataacaa tttcacacag gaaacagaat tcccagcttg gctgcagaac 3300
cattccattc gttgatccgg gagtaactca c 3331

<210> 2
<211> 771
<212> DNA
<213> Treponema pallidum

<220>
<221> CDS
<222> (1)..(771)

<400> 2
atg aaa ctg aag gta aca gtc aac ggc act gcg tat gac gtt gac gtt 48
Met Lys Leu Lys Val Thr Val Asn Gly Thr Ala Tyr Asp Val Asp Val
1               5                   10                  15

gac gtc gac aag tca cac gaa aac ccg atg ggc acc atc ctg ttc ggc 96
Asp Val Asp Lys Ser His Glu Asn Pro Met Gly Thr Ile Leu Phe Gly
            20                  25                  30

ggc ggc acc ggc ggc gcg ccg gca ccg gca gca ggt ggc gca ggc gcc 144
Gly Gly Thr Gly Gly Ala Pro Ala Pro Ala Ala Gly Gly Ala Gly Ala
        35                  40                  45

ggt aag gcc gga gag ggc gag att ccc gct ccg ctg gcc ggc acc gtc 192
Gly Lys Ala Gly Glu Gly Glu Ile Pro Ala Pro Leu Ala Gly Thr Val
    50                  55                  60

tcc aag atc ctc gtg aag gag ggt gac acg gtc aag gct ggt cag acc 240
Ser Lys Ile Leu Val Lys Glu Gly Asp Thr Val Lys Ala Gly Gln Thr
65                  70                  75                  80

gtg ctc gtt ctc gag gcc atg aag atg gag acc gag atc aac gct ccc 288
Val Leu Val Leu Glu Ala Met Lys Met Glu Thr Glu Ile Asn Ala Pro
                85                  90                  95

acc gac ggc aag gtc gag aag gtc ctg gtc aag gag cgt gac gcg gtg 336
Thr Asp Gly Lys Val Glu Lys Val Leu Val Lys Glu Arg Asp Ala Val
            100                 105                 110

cag ggc ggt cag ggt ctc atc aag atc ggg gat ctc gag ctc atc gaa 384
Gln Gly Gly Gln Gly Leu Ile Lys Ile Gly Asp Leu Glu Leu Ile Glu
        115                 120                 125

ggt cgc gaa aag ctt tct ttc agt tca att cca aat ggc acg tac cgg 432
Gly Arg Glu Lys Leu Ser Phe Ser Ser Ile Pro Asn Gly Thr Tyr Arg
    130                 135                 140

gcg acg tat cag gat ttt gat gag aat ggt tgg aag gac ttt ctc gag 480
Ala Thr Tyr Gln Asp Phe Asp Glu Asn Gly Trp Lys Asp Phe Leu Glu
145                 150                 155                 160

gtt act ttt gat ggt ggc aag atg gtg cag gtg gtt tac gat tat cag 528
Val Thr Phe Asp Gly Gly Lys Met Val Gln Val Val Tyr Asp Tyr Gln
                165                 170                 175

cat aaa gaa ggg cgg ttt aag tcc cag gac gct gac tac cat cgg gtc 576
His Lys Glu Gly Arg Phe Lys Ser Gln Asp Ala Asp Tyr His Arg Val
            180                 185                 190

atg tat gca tcc tcg ggc ata ggt cct gaa aag gcc ttc aga gag ctc 624
Met Tyr Ala Ser Ser Gly Ile Gly Pro Glu Lys Ala Phe Arg Glu Leu
        195                 200                 205

gcc gat gct ttg ctt gaa aag ggt aat ccc gag atg gtg gat gtg gtc 672
Ala Asp Ala Leu Leu Glu Lys Gly Asn Pro Glu Met Val Asp Val Val
    210                 215                 220

acc ggt gca act gtt tct tcc cag agt ttc agg agg ttg ggt cgt gcg 720
Thr Gly Ala Thr Val Ser Ser Gln Ser Phe Arg Arg Leu Gly Arg Ala
225                 230                 235                 240

ctt ctg cag agt gcg cgg cgc ggc gag aag gaa gcc att att agc agg 768
Leu Leu Gln Ser Ala Arg Arg Gly Glu Lys Glu Ala Ile Ile Ser Arg
                245                 250                 255

tag 771

<210> 3
<211> 256
<212> PRT
<213> Treponema pallidum

<400> 3
Met Lys Leu Lys Val Thr Val Asn Gly Thr Ala Tyr Asp Val Asp Val
1               5                   10                  15

Asp Val Asp Lys Ser His Glu Asn Pro Met Gly Thr Ile Leu Phe Gly
            20                  25                  30

Gly Gly Thr Gly Gly Ala Pro Ala Pro Ala Ala Gly Gly Ala Gly Ala
        35                  40                  45

Gly Lys Ala Gly Glu Gly Glu Ile Pro Ala Pro Leu Ala Gly Thr Val
    50                  55                  60

Ser Lys Ile Leu Val Lys Glu Gly Asp Thr Val Lys Ala Gly Gln Thr
65                  70                  75                  80

Val Leu Val Leu Glu Ala Met Lys Met Glu Thr Glu Ile Asn Ala Pro
                85                  90                  95

Thr Asp Gly Lys Val Glu Lys Val Leu Val Lys Glu Arg Asp Ala Val
            100                 105                 110

Gln Gly Gly Gln Gly Leu Ile Lys Ile Gly Asp Leu Glu Leu Ile Glu
        115                 120                 125

Gly Arg Glu Lys Leu Ser Phe Ser Ser Ile Pro Asn Gly Thr Tyr Arg
    130                 135                 140

Ala Thr Tyr Gln Asp Phe Asp Glu Asn Gly Trp Lys Asp Phe Leu Glu
145                 150                 155                 160

Val Thr Phe Asp Gly Gly Lys Met Val Gln Val Val Tyr Asp Tyr Gln
                165                 170                 175

His Lys Glu Gly Arg Phe Lys Ser Gln Asp Ala Asp Tyr His Arg Val
            180                 185                 190

Met Tyr Ala Ser Ser Gly Ile Gly Pro Glu Lys Ala Phe Arg Glu Leu
        195                 200                 205

Ala Asp Ala Leu Leu Glu Lys Gly Asn Pro Glu Met Val Asp Val Val
    210                 215                 220

Thr Gly Ala Thr Val Ser Ser Gln Ser Phe Arg Arg Leu Gly Arg Ala
225                 230                 235                 240

Leu Leu Gln Ser Ala Arg Arg Gly Glu Lys Glu Ala Ile Ile Ser Arg
                245                 250                 255

<210> 4
<211> 378
<212> DNA
<213> Treponema pallidum

<220>
<221> CDS
<222> (1)..(378)

<400> 4

atg aaa ctg aag gta aca gtc aac ggc act gcg tat gac gtt gac gtt     48
Met Lys Leu Lys Val Thr Val Asn Gly Thr Ala Tyr Asp Val Asp Val
  1               5                  10                  15

gac gtc gac aag tca cac gaa aac ccg atg ggc acc atc ctg ttc ggc     96
Asp Val Asp Lys Ser His Glu Asn Pro Met Gly Thr Ile Leu Phe Gly
             20                  25                  30

ggc ggc acc ggc ggc gcg ccg gca ccg gca gca ggt ggc gca ggc gcc     144
Gly Gly Thr Gly Gly Ala Pro Ala Pro Ala Ala Gly Gly Ala Gly Ala
         35                  40                  45

ggt aag gcc gga gag ggc gag att ccc gct ccg ctg gcc ggc acc gtc     192
Gly Lys Ala Gly Glu Gly Glu Ile Pro Ala Pro Leu Ala Gly Thr Val
     50                  55                  60

tcc aag atc ctc gtg aag gag ggt gac acg gtc aag gct ggt cag acc     240
Ser Lys Ile Leu Val Lys Glu Gly Asp Thr Val Lys Ala Gly Gln Thr
 65                  70                  75                  80

gtg ctc gtt ctc gag gcc atg aag atg gag acc gag atc aac gct ccc     288
Val Leu Val Leu Glu Ala Met Lys Met Glu Thr Glu Ile Asn Ala Pro
                 85                  90                  95

acc gac ggc aag gtc gag aag gtc ctg gtc aag gag cgt gac gcg gtg     336
Thr Asp Gly Lys Val Glu Lys Val Leu Val Lys Glu Arg Asp Ala Val
            100                 105                 110

cag ggc ggt cag ggt ctc atc aag atc ggg gat ctc gag ctc             378
Gln Gly Gly Gln Gly Leu Ile Lys Ile Gly Asp Leu Glu Leu
        115                 120                 125

<210> 5
<211> 126
<212> PRT
<213> Treponema pallidum

<400> 5
Met Lys Leu Lys Val Thr Val Asn Gly Thr Ala Tyr Asp Val Asp Val
  1               5                  10                  15

Asp Val Asp Lys Ser His Glu Asn Pro Met Gly Thr Ile Leu Phe Gly
             20                  25                  30

Gly Gly Thr Gly Gly Ala Pro Ala Pro Ala Ala Gly Gly Ala Gly Ala
         35                  40                  45

Gly Lys Ala Gly Glu Gly Glu Ile Pro Ala Pro Leu Ala Gly Thr Val
     50                  55                  60

Ser Lys Ile Leu Val Lys Glu Gly Asp Thr Val Lys Ala Gly Gln Thr
 65                  70                  75                  80

Val Leu Val Leu Glu Ala Met Lys Met Glu Thr Glu Ile Asn Ala Pro
                 85                  90                  95

Thr Asp Gly Lys Val Glu Lys Val Leu Val Lys Glu Arg Asp Ala Val
            100                 105                 110

Gln Gly Gly Gln Gly Leu Ile Lys Ile Gly Asp Leu Glu Leu
        115                 120                 125

<210> 6
<211> 801
<212> DNA
<213> Treponema pallidum

<220>
<221> CDS
<222> (1)..(801)

<400> 6
atg aaa ctg aag gta aca gtc aac ggc act gcg tat gac gtt gac gtt      48
Met Lys Leu Lys Val Thr Val Asn Gly Thr Ala Tyr Asp Val Asp Val
  1               5                  10                  15

gac gtc gac aag tca cac gaa aac ccg atg ggc acc atc ctg ttc ggc      96
Asp Val Asp Lys Ser His Glu Asn Pro Met Gly Thr Ile Leu Phe Gly
             20                  25                  30

ggc ggc acc ggc ggc gcg ccg gca ccg gca gca ggt ggc gca ggc gcc     144
Gly Gly Thr Gly Gly Ala Pro Ala Pro Ala Ala Gly Gly Ala Gly Ala
         35                  40                  45

ggt aag gcc gga gag ggc gag att ccc gct ccg ctg gcc ggc acc gtc     192
Gly Lys Ala Gly Glu Gly Glu Ile Pro Ala Pro Leu Ala Gly Thr Val
     50                  55                  60

tcc aag atc ctc gtg aag gag ggt gac acg gtc aag gct ggt cag acc     240
Ser Lys Ile Leu Val Lys Glu Gly Asp Thr Val Lys Ala Gly Gln Thr
 65                  70                  75                  80

gtg ctc gtt ctc gag gcc atg aag atg gag acc gag atc aac gct ccc     288
Val Leu Val Leu Glu Ala Met Lys Met Glu Thr Glu Ile Asn Ala Pro
                 85                  90                  95

acc gac ggc aag gtc gag aag gtc ctg gtc aag gag cgt gac gcg gtg     336
Thr Asp Gly Lys Val Glu Lys Val Leu Val Lys Glu Arg Asp Ala Val
            100                 105                 110

cag ggc ggt cag ggt ctc atc aag atc ggg gat ctc gag ctc atc gaa     384
Gln Gly Gly Gln Gly Leu Ile Lys Ile Gly Asp Leu Glu Leu Ile Glu
        115                 120                 125

ggt cgc gaa aag ctt tca tgt aca acc gtg tgt ccg cac gcc ggg aag     432
Gly Arg Glu Lys Leu Ser Cys Thr Thr Val Cys Pro His Ala Gly Lys
    130                 135                 140

gcc aaa gcg gaa aag gta gag tgc gcg ttg aag gga ggt atc ttt cgg     480
Ala Lys Ala Glu Lys Val Glu Cys Ala Leu Lys Gly Gly Ile Phe Arg
145                 150                 155                 160

ggt acg cta cct gcg gcc gat tgc ccg gga atc gat acg act gtg acg     528
Gly Thr Leu Pro Ala Ala Asp Cys Pro Gly Ile Asp Thr Thr Val Thr
                165                 170                 175

ttc aac gcg gat ggc act gcg caa aag gta gag ctt gcc ctt gag aag     576
Phe Asn Ala Asp Gly Thr Ala Gln Lys Val Glu Leu Ala Leu Glu Lys
            180                 185                 190

aag tcg gca cct tct cct ctt acc tat cgc ggt acg tgg atg gta cgt     624
Lys Ser Ala Pro Ser Pro Leu Thr Tyr Arg Gly Thr Trp Met Val Arg
        195                 200                 205

gaa gac gga att gtc gaa ctc tcg ctt gtg tcc tcg gag caa tcg aag     672
Glu Asp Gly Ile Val Glu Leu Ser Leu Val Ser Ser Glu Gln Ser Lys
    210                 215                 220

gca ccg cac gag aaa gag ctg tac gag ctg ata gac agt aac tcc gtt     720
Ala Pro His Glu Lys Glu Leu Tyr Glu Leu Ile Asp Ser Asn Ser Val
225                 230                 235                 240

cgc tac atg ggc gct ccc ggc gca gga aag cct tca aag gag atg gcg     768
Arg Tyr Met Gly Ala Pro Gly Ala Gly Lys Pro Ser Lys Glu Met Ala
                245                 250                 255

ccg ttt tac gtg ctc aaa aaa aca aag aaa tag                         801
Pro Phe Tyr Val Leu Lys Lys Thr Lys Lys
            260                 265

<210> 7
<211> 266
<212> PRT
<213> Treponema pallidum

<400> 7
Met Lys Leu Lys Val Thr Val Asn Gly Thr Ala Tyr Asp Val Asp Val
1               5                   10                  15

Asp Val Asp Lys Ser His Glu Asn Pro Met Gly Thr Ile Leu Phe Gly
            20                  25                  30

Gly Gly Thr Gly Gly Ala Pro Ala Pro Ala Ala Gly Gly Ala Gly Ala
        35                  40                  45

Gly Lys Ala Gly Glu Gly Glu Ile Pro Ala Pro Leu Ala Gly Thr Val
    50                  55                  60

Ser Lys Ile Leu Val Lys Glu Gly Asp Thr Val Lys Ala Gly Gln Thr
65                  70                  75                  80

Val Leu Val Leu Glu Ala Met Lys Met Glu Thr Glu Ile Asn Ala Pro
                85                  90                  95

Thr Asp Gly Lys Val Glu Lys Val Leu Val Lys Glu Arg Asp Ala Val
            100                 105                 110

Gln Gly Gly Gln Gly Leu Ile Lys Ile Gly Asp Leu Glu Leu Ile Glu
        115                 120                 125

Gly Arg Glu Lys Leu Ser Cys Thr Thr Val Cys Pro His Ala Gly Lys
    130                 135                 140

Ala Lys Ala Glu Lys Val Glu Cys Ala Leu Lys Gly Gly Ile Phe Arg
145                 150                 155                 160

Gly Thr Leu Pro Ala Ala Asp Cys Pro Gly Ile Asp Thr Thr Val Thr
                165                 170                 175

Phe Asn Ala Asp Gly Thr Ala Gln Lys Val Glu Leu Ala Leu Glu Lys
            180                 185                 190

Lys Ser Ala Pro Ser Pro Leu Thr Tyr Arg Gly Thr Trp Met Val Arg
        195                 200                 205

Glu Asp Gly Ile Val Glu Leu Ser Leu Val Ser Ser Glu Gln Ser Lys
    210                 215                 220

Ala Pro His Glu Lys Glu Leu Tyr Glu Leu Ile Asp Ser Asn Ser Val
225                 230                 235                 240

Arg Tyr Met Gly Ala Pro Gly Ala Gly Lys Pro Ser Lys Glu Met Ala
                245                 250                 255

Pro Phe Tyr Val Leu Lys Lys Thr Lys Lys
            260                 265